(19)

Europäisches
Patentamt
European
Patent Office
Office européen
des brevets

(11) **EP 2 420 843 A1**

(12) **EUROPEAN PATENT APPLICATION**

(43) Date of publication:
**22.02.2012 Bulletin 2012/08**

(51) Int Cl.:
**G01N 33/68** [(2006.01)]

(21) Application number: **10172846.7**

(22) Date of filing: **13.08.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicants:
• **Universiteit Maastricht**
  **6211 LK  Maastricht (NL)**
• **Academisch Ziekenhuis Maastricht**
  **6229 HX Maastricht (NL)**

(72) Inventors:
• **Wang, Ping**
  **6200 MD Maastricht (NL)**
• **Mariman, Edwin Cyriel Maria**
  **6200 MD Maastricht (NL)**

(74) Representative: **Habets, Winand**
  **Life Science Patents**
  **PO Box 5096**
  **6130 PB  Sittard (NL)**

(54) **Biomarkers for predicting maintenance of weight loss**

(57) The invention is in the field of medical and cosmetic diagnostics. It provides methods for determining the probability that a person will maintain weight loss after an intentional weight loss. In particular, the invention provides such a method by determining the level of angiotensin I converting enzyme (ACE) in a first sample obtained before the start of the diet and in a second sample obtained after the dietary period wherein the ratio of ACE levels in the second sample and the first sample is determined and compared to a predetermined reference value and wherein it is concluded that the individual has a higher probability to be successful in maintaining weight loss if said ratio is below the predetermined reference value.

Figure 1

Number of test persons

EP 2 420 843 A1

**Description**

<u>Field of the invention</u>

The invention is in the field of medical and cosmetic diagnostics. It provides methods for determining the probability that a person will maintain weight loss after a weight loss due to a dietary period.

<u>Background of the invention</u>

The worldwide epidemic of obesity and related health problems like diabetes (1) and others including cardiovascular disorders and certain types of cancer demands effective measures to help overweight and obese people to reduce their weight. However, to maintain a reduced weight is a big challenge because a majority of the people regains weight on the long term (2).

It is well recognised that obesity has both a genetic and an environmental basis. An individual's susceptibility is determined largely by genetics, while the observed outcome is determined by environmental factors (diet, physical activity, etc.). In addition to a single-gene variant causing obesity (5), there is growing evidence that gene-environment interactions influence the development of obesity and the weight change by intervention (6-8). The success of a dietary intervention on weight maintenance can be influenced by the genetic background of the subject.

The development of obesity is a complex physiological process, so are weight loss and weight maintenance.

It has been suggested in the literature that low calorie/high protein diets are more likely to prevent weight gain after initial weight loss. Others recommend exercise as the remedy against weight gain after initial weight loss. It has also been observed that people that received counseling by either a dietitian or a psychologist were less prone to weight gain than the control group which did not receive such counseling. These factors have been reviewed in references 10 and 11.

Biomarkers that have been suggested for predicting maintaining successful weight loss are for instance genetic polymorphisms (7, 9). Detection of such polymorphisms however, requires extensive and expensive analyses.

Despite of these achievements, there remains a need in the art for reliable and easy-to-measure biomarkers for predicting whether an initial weight loss can be successfully maintained.

<u>Summary of the invention</u>

It was found that certain biomarkers could reliably predict whether weight loss after a dietary period could be successfully maintained.

Hence, the invention relates to a method for determining the probability that an individual will be successful in maintaining weight loss after an intentional weight loss by determining the level of angiotensin I converting enzyme (ACE) in a first sample obtained before the start of the weight loss and in a second sample obtained after the weight loss wherein the ratio of ACE levels in the second sample and the first sample is determined and compared to a first predetermined reference value and wherein it is concluded that the individual has a higher probability to be successful in maintaining weight loss if said ratio is lower than the first predetermined reference value.

It was also found that this method could be improved by including a second biomarker into the above method. The invention therefore also relates to a method as described above wherein the first sample obtained from the individual has a high fibrinogen level.

<u>Detailed description of the invention</u>

The relative efficacy in weight maintenance after weight loss of four different diets with a variation in protein/carbohydrate content and glycemic index (GI) was investigated in obese/overweight European families (3, 4).

Our study cohort consisted of 96 overweight individuals, selected as described in example 1. In brief, families with at least one overweight or obese but otherwise healthy parent were recruited. From clinical investigation day 1 (CID1), participants started an 8-week low calorie diet (LCD). Families with overweight/obese parent(s) that achieved ≥8% of initial body weight loss during LCD were randomized to one of 4 low fat ad libitum diets for a 6-month weight maintenance period with dietary counseling every 2-4 weeks.

Angiotensin I converting enzyme (ACE) levels were determined in samples obtained at the beginning of the study and at the end of the LCD period (CID2). It appeared that the ratio of ACE levels at CID2 and CID1 could reliably predict whether weight loss after a dietary period could be successfully maintained.

Hence, the invention relates to a method for determining the probability that an individual will be successful in maintaining weight loss after an intentional weight loss by determining the level of angiotensin I converting enzyme (ACE) in a first sample obtained before the start of the weight loss and in a second sample obtained after the weight loss wherein the ratio of ACE levels in the second sample and the first sample is determined and compared to a first predetermined reference value and wherein it is concluded that the individual has a higher probability to be successful in maintaining weight loss if said ratio is higher than the first predetermined reference value.

The term "successful in maintaining weight loss" in this context means that an individual who intentionally lost weight due to a dietary period does not regain more than 25% of the weight that he or she has lost, in a period of 6 months after the end of the dietary period. Preferably not more than 15% of the lost weight should be regained, such as 12%, 10%, 8%, 6%, 4% 2% or even 0%. It is also conceivable that the subject looses further weight, such individuals are also comprised in the term successful maintenance of weight loss.

The term "sample" is to be interpreted as a sample obtained from a subject, preferably isolated from that subject wherein the step comprising the isolating or obtaining from the subject may not be an integral part of the method according to the invention. The sample may be any sample obtained from the subject such as urine, saliva, sweat, vaginal secretions, sperm, swaps from mucosal tissue, blood, serum or plasma. A sample is preferably a serum or plasma sample.

The term "intentional weight loss" refers to a weight loss due to an action undertaken by a subject intended to lose weight. Such action may be for example exercise, fasting, diet or medical treatment. Preferably, the invention relates to a method as described above wherein the intentional weight loss is due to a dietary period.

Hence, the invention also relates to a method for determining the probability that an individual will be successful in maintaining weight loss after a weight loss due to a dietary period by determining the level of angiotensin I converting enzyme (ACE) in a first sample obtained before the start of the diet and in a second sample obtained after the dietary period wherein the ratio of ACE levels in the second sample and the first sample is determined and compared to a first predetermined reference value and wherein it is concluded that the individual has a higher probability to be successful in maintaining weight loss if said ratio is higher than the first predetermined reference value.

The term "dietary weight loss" or "weight loss due to a dietary period" is to be interpreted as the weight loss due to a diet, preferably a low calorie diet. Weight loss is to be calculated as the weight at the onset of the dietary period minus the weight at the end of the dietary period.

The term "dietary period" means the time span wherein a diet is taken, usually a number of weeks, such as 2, 3, 4, 5, 6, 7, 8 or more weeks, up to 10 or 20 weeks..

The level of angiotensin I converting enzyme (ACE) is an art-recognized term and it may be determined by any suitable method known in the art. For example by the commercially available radiochemical (RC) assay (Ventrex®) and a spectrophotometric (SP) assay for serum angiotensin-converting enzyme. Other methods may be equally suited.

The term "first predetermined reference value" in this context is usually also referred to as cut-off value and means a value empirically determined or calculated to distinguish the positive from the negative values, or as the case may be, the high from the low values. A reference value may be an average, mean or median value obtained from observations or measurements in a normal population or in a population lacking the feature the test is meant to measure. More in particular, in this case the reference value for determining the ratio of ACE values was set at 0.85. If an ACE ratio was found to be lower than this particular reference value, the individual had a high probability of successfully maintaining the weight loss of the dietary period. On the other hand, if the ACE ratio was above a predetermined reference value of 0.95, the individual had a low chance of successfully maintaining the weight loss. Preferably, the first predetermined reference value is the mean ratio of CID2/CID1 of a population with comparable anthropometric and physiological characteristics. Preferably, such characteristics include include BMI and/or cholesterol levels.

The first predetermined reference value may vary depending on the particular methods used to determine the ACE values and ratios, however, it is within the reach of a skilled person to determine that ratio with the guidance provided herein, in particular the experiments described in the Examples section

It was also found that this method could be improved by including a second biomarker into the above method. It was observed that 51 persons had a high fibrinogen level at the onset of the study. Within this group of individuals with a high fibrinogen level, the CID2/CID1 ratio of ACE levels even more reliably predicted the outcome of successful weight maintenance.

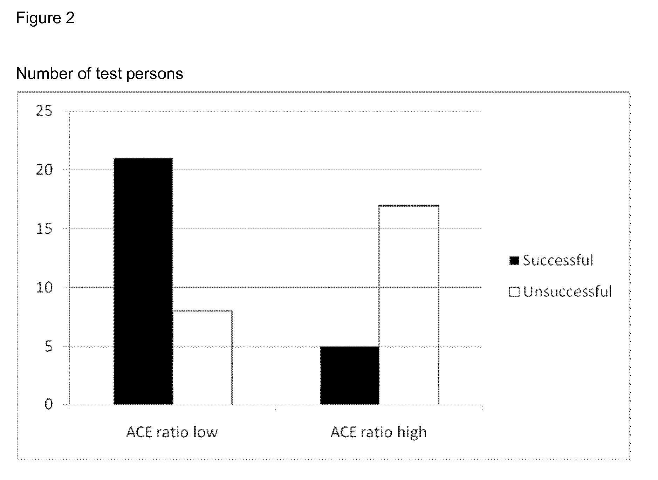

Hence, it appeared that the ratio of ACE levels in combination with fibrinogen level at the onset of the study reliably predicted whether weight loss was successful or not. This was even more reliable than the methods based on the ratio of ACE levels alone. The ACE ratios in the group of individuals with a high fibrinogen level are shown in table 1 and figure 2.

Table 1. Distribution of successful and unsuccessful outcome of weight maintenance in the group with high levels of fibrinogen divided over high and low ACE CID2/CIDI ratios.

|  | ACE CID2/CIDI ratio low, high fibrinogen | ACE CID2/CIDI ratio high, high fibrinogen |
|---|---|---|
| Successful | 21 | 5 |

(continued)

| | ACE CID2/CIDI ratio low, high fibrinogen | ACE CID2/CIDI ratio high, high fibrinogen |
|---|---|---|
| Unsuccessful | 8 | 17 |

Fibrinogen levels at the onset of the study were as such not predictive of successful or unsuccessful weight maintenance after weight loss (see Table 2).

Table 2 Fibrinogen levels in plasma (mean $\pm$ SD)

| | | | **CID1** |
|---|---|---|---|
| pool | Successful | N=47 | 9.87 $\pm$ 1.75 |
| | Unsuccessful | N=48 | 9.47 $\pm$ 2.32 |
| Diet1 LP/LGI | Successful | N=11 | 9.64 $\pm$ 2.05 |
| | Unsuccessful | N=12 | 9.85 $\pm$ 2.33 |
| Diet2 LP/HGI | Successful | N=12 | 9.61 $\pm$ 1.52 |
| | Unsuccessful | N=12 | 9.96 $\pm$ 1.99 |
| Diet3 HP/LGI | Successful | N=12 | 10.04 $\pm$ 1.93 |
| | Unsuccessful | N=12 | 9.47 $\pm$ 1.94 |
| Diet4 HP/HGI | Successful | N=12 | 10.16 $\pm$ 1.64 |
| | Unsuccessful | N=12 | 8.59 $\pm$ 2.94 |

The invention therefore also relates to a method as described above wherein a sample obtained from the individual at the onset of the study, i.e. before weight loss, has a fibrinogen level above a predetermined reference value.

The term "high ratio" or "high value" or equivalent expressions are meant to indicate that a ratio or a value is above a certain reference value. The term "low ratio" or "low value" is meant to indicate a ratio or value below a certain reference value.

The term a "high fibrinogen level" means a fibrinogen level above a predetermined reference value. Preferably this reference value is obtained from a group of reference subjects comparable with respect to anthropometric and physiological characteristics. Preferably, the reference value is the mean or median of the fibrinogen levels in these reference subjects. Preferably, the anthropometric and physiological characteristics of the reference subjects include BMI and/or cholesterol levels.

It is preferred to use subjects with comparable BMI or cholesterol levels as reference subjects. Comparable in this context means within a range of 20%, such as 15%, 10% and 5%.

Legend to the figures

Figure 1: Number of persons with successful and unsuccessful weight maintenance after the diets. Those individuals with a low ACE ratio CID2/CIDI had a higher chance of being successful in maintaining their weight than the group with a high ACE ratio CID2/CID1.

Figure 2: Number of persons with a high fibrinogen level at the onset of the study were divided into two groups, one with successful and the other with unsuccessful weight maintenance after the diets. Those individuals with a high fibrinogen level and a low ACE ratio CID2/CIDl had a higher chance of being successful in maintaining their weight than the group with a high fibrinogen level and a high ACE ratio CID2/CID1.

Figure 3: Graph wherein the probability of success obtained with the method for predicting successful weight maintenance is plotted against the CID2/CID1 ratio of ACE.

Examples

Example 1: Subjects and study design

Subjects were participants in the Diogenes study. This study is a pan-European, randomized and controlled dietary intervention study (3, 4). The study was approved by local ethical committees in the respective countries and all participants signed an informed consent. In brief, families with at least one overweight or obese but otherwise healthy parent were recruited. From clinical investigation day 1 (CID1), participants started an 8-week low calorie diet (LCD). After undergoing clinical investigation, 8 weeks after the start of the trial (at CID2) those families with overweight/obese parent(s) that achieved ≥8% of initial body weight loss during LCD were randomized to one of the following 4 low fat ad libitum diets or a control diet for a 6-month weight maintenance period with dietary counseling every 2-4 weeks (3): Diet1: low protein (LP) and low glycemic index (LGI), Diet2: LP and high glycemic index (HGI), Diet3: high protein (HP) and LGI, Diet4: HP and HGI.

After this intervention period of 6 months (CID3), the participants underwent clinical investigation again. On each CID, the anthropometric and physiological parameters were measured, and blood, urine and fat biopsy samples were collected (4). The collecting at each centre was performed using the same standardized protocol. Fasting EDTA plasma and serum samples were aliquoted and kept at -80˚C during storage and transportation. In addition, blood glucose, triglycerides, cholesterols and other factors were measured (4).

In one study, we focused on female participants of the 4 intervention diets. There were 236 adult Caucasian females who completed the dietary intervention and were below 50 years old, non-diabetic and non-dyslipidemic with fasting glucose < 7mM, triglyceride < 3.6mM, total cholesterol < 7mM at CID1. An 's-value' was used to assess the outcome of weight maintenance. It is the weight change during the weight maintenance phase divided by the weight loss during the weight loss phase. In this way the influence of weight loss on weight maintenance is taken into account.

$$s = (\text{body weight at CID3} - \text{body weight at CID2}) / (\text{body weight at CID 1} - \text{body weight at CID 2})$$

The subjects beyond the 10-90 percentiles of the s-value of each diet group were taken as the extremes and excluded. From the remaining subjects for each diet, the 12 with the lowest s-value were defined as the successful group and the 12 with highest as the unsuccessful group. In total, 96 subjects were selected as our study cohort.

The anthropometric and physiological characteristics are listed in **Table 3**. The group of 48 successful subjects lost 3.3±2.2 kg weight at CID3 while the group of 48 unsuccessful subjects regained 3.9±1.2 kg weight during the 6-month maintenance period. The anthropometric and physiological characteristics of the groups of successful and unsuccessful subjects were comparable at baseline (CID1) and after weight loss (CID2) overall.

Table 3 Anthropometric and physiological characteristics of patient population in this study

| | CID | Pool Success N=48 | Pool Unsuccess N=48 | Diet1 LP/LGI Success N=12 | Diet1 LP/LGI Unsuccess N=12 | Diet2 LP/HGI Success N=12 | Diet2 LP/HGI Unsuccess N=12 | Diet3 HP/LGI Success N=12 | Diet3 HP/LGI Unsuccess N=12 | Diet4 HP/HGI Success N=12 | Diet4 HP/HGI Unsuccess N=12 |
|---|---|---|---|---|---|---|---|---|---|---|---|
| Weight maintenance score | | -0.28 ± 0.14 | 0.42 ± 0.12 | -0.31 ± 0.11 | 0.43 ± 0.10 | -0.17 ± 0.10 | 0.52 ± 0.11 | -0.36 ± 0.20 | 0.38 ± 0.11 | -0.30 ± 0.08 | 0.33 ± 0.10 |
| Age (year) | | 39.3 ± 5.3 | 41.3 ± 5.0 | 43.0 ± 5.8 | 41.1 ± 3.8 | 38.3 ± 3.6 | 42.9 ± 3.6 | 38.4 ± 4.4 | 39.3 ± 6.0 | 37.4 ± 5.6 | 41.8 ± 6.0 |
| Weight (kg) | CID1 | 97.5 ± 16.4[a] | 93.3 ± 12.6[a] | 101.3 ± 18.5[a] | 98.9 ± 10.8[a] | 87.3 ± 6.9[a] | 91.9 ± 12.8[a] | 104.5 ± 16.4[a] | 91.6 ± 16.2[a] | 96.7 ± 17.3[a] | 91.0 ± 9.3[a] |
| Weight (kg) | CID2 | 86.3 ± 14.6[b] | 83.8 ± 11.8[b] | 89.7 ± 17.5[b] | 88.7 ± 9.8[b] | 78.0 ± 7.6[b] | 83.1 ± 12.6[b] | 91.7 ± 14.7[b] | 81.7 ± 15.3[b] | 85.8 ± 14.4[b] | 81.5 ± 8.4[b] |
| Weight (kg) | CID3 | 83.0 ± 13.6[c] | 87.7 ± 12.2[c] | 86.1 ± 16.9[c] | 93.2 ± 10.4[c] | 76.5 ± 7.8[c] | 87.5 ± 12.5[c] | 87.1 ± 13.5[c] | 85.4 ± 15.9[c] | 82.4 ± 13.6[c] | 84.7 ± 8.2[c] |
| BMI (kg·m$^{-2}$) | CID1 | 34.5 ± 4.8[a] | 33.3 ± 4.3[a] | 35.8 ± 5.0[a] | 34.6 ± 4.4[a] | 30.9 ± 2.6[a] | 33.4 ± 4.4[a] | 36.3 ± 4.2[a] | 32.2 ± 4.5[a] | 35.0 ± 5.4[a] | 33.0 ± 4.2[a] |
| BMI (kg·m$^{-2}$) | CID2 | 30.6 ± 4.3[b] | 29.9 ± 4.1[b] | 31.6 ± 4.9[b] | 31.1 ± 4.2[b] | 27.6 ± 2.9[b] | 30.2 ± 4.4[b] | 31.9 ± 3.8[b] | 28.8 ± 4.2[b] | 31.1 ± 4.5[b] | 29.5 ± 3.7[b] |
| BMI (kg·m$^{-2}$) | CID3 | 29.4 ± 4.0[c] | 31.3 ± 4.2[c] | 30.4 ± 4.8[c] | 32.6 ± 4.3[c] | 27.1 ± 2.9[c] | 31.8 ± 4.5[c] | 30.3 ± 3.3[c] | 30.0 ± 4.3[c] | 29.8 ± 4.2[c] | 30.6 ± 3.7[c] |
| Waist (cm) | CID1 | 104 ± 13 *** | 102 ± 11 *** | 107 ± 13 *** | 107 ± 10 *** | 96 ± 7 *** | 101 ± 13 ** | 107 ± 9 *** | 101 ± 13 *** | 105 ± 19 ** | 99 ± 10 *** |
| Waist (cm) | CID2 | 95 ± 12 | 94 ± 10 | 96 ± 12 | 98 ± 9 | 86 ± 7 | 94 ± 9 | 100 ± 7 | 92 ± 13 | 96 ± 16 | 93 ± 9 |
| Systolic blood pressure (mmHg) | CID1 | 121 ± 13 ** | 120 ± 12 ** | 128 ± 12 | 122 ± 13 * | 114 ± 10 | 116 ± 13 | 116 ± 12 | 119 ± 11 | 124 ± 12 | 124 ± 13 * |
| Systolic blood pressure (mmHg) | CID2 | 116 ± 12 | 114 ± 13 | 123 ± 12 | 115 ± 12 | 110 ± 11 | 110 ± 15 | 113 ± 10 | 118 ± 11 | 119 ± 14 | 114 ± 13 |
| Diastolic blood pressure (mmHg) | CID1 | 75 ± 10 ** | 73 ± 10 | 80 ± 11 * | 75 ± 9 | 71 ± 8 | 70 ± 12 | 74 ± 6 | 73 ± 11 | 75 ± 13 | 75 ± 8 |
| Diastolic blood pressure (mmHg) | CID2 | 71 ± 9 | 71 ± 11 | 73 ± 7 | 74 ± 12 | 69 ± 11 | 68 ± 8 | 70 ± 8 | 75 ± 12 | 73 ± 11 | 69 ± 10 |
| Cholesterol (mmol·L-1) | CID1 | 4.6 ± 0.8 *** | 4.6 ± 1.0 ** | 5.0 ± 0.8 * | 4.3 ± 1.4 | 4.6 ± 0.8 ** | 4.9 ± 0.9 * | 4.5 ± 0.9 | 4.6 ± 0.7 ** | 4.5 ± 0.7 ** | 4.7 ± 1.0 |
| Cholesterol (mmol·L-1) | CID2 | 4.1 ± 0.7 | 4.2 ± 0.8 | 4.3 ± 0.8 | 4.0 ± 1.0 | 3.9 ± 0.4 | 4.4 ± 0.6 | 4.1 ± 0.4 | 4.0 ± 0.9 | 4.0 ± 0.9 | 4.2 ± 0.8 |
| Triglycerides (mmol·L-1) | CID1 | 1.3 ± 0.5 ** | 1.2 ± 0.5 | 1.4 ± 0.6 * | 1.2 ± 0.6 | 1.1 ± 0.4 | 1.1 ± 0.6 | 1.2 ± 0.7 | 1.0 ± 0.4 | 1.2 ± 0.3 | 1.5 ± 0.5 |
| Triglycerides (mmol·L-1) | CID2 | 1.1 ± 0.3 | 1.1 ± 0.5 | 1.1 ± 0.4 | 1.2 ± 0.8 | 1.0 ± 0.4 | 1.0 ± 0.4 | 1.1 ± 0.3 | 0.9 ± 0.3 | 1.1 ± 0.3 | 1.3 ± 0.4 |

| | | Pool | | | | Diet1 LP/LGI | | | | Diet2 LP/HGI | | | | Diet3 HP/LGI | | | | Diet4 HP/HGI | | | |
|---|---|---|---|---|---|---|---|---|---|---|---|---|---|---|---|---|---|---|---|---|---|
| | | Success N=48 | | Unsuccess N=48 | | Success N=12 | | Unsuccess N=12 | | Success N=12 | | Unsuccess N=12 | | Success N=12 | | Unsuccess N=12 | | Success N=12 | | Unsuccess N=12 | |
| HDL | CID1 | 1.3 ± 0.3 | ** | 1.3 ± 0.4 | * | 1.3 ± 0.3 | | 1.1 ± 0.4 | | 1.3 ± 0.3 | * | 1.4 ± 0.3 | | 1.3 ± 0.3 | | 1.4 ± 0.4 | | 1.2 ± 0.2 | | 1.1 ± 0.3 | |
| (mmol·L-1) | CID2 | 1.2 ± 0.2 | | 1.2 ± 0.3 | | 1.2 ± 0.3 | | 1.1 ± 0.3 | | 1.2 ± 0.3 | | 1.3 ± 0.2 | | 1.2 ± 0.2 | | 1.3 ± 0.4 | | 1.1 ± 0.2 | | 1.1 ± 0.2 | |
| LDL | CID1 | 2.8 ± 0.7 | ** | 2.8 ± 0.8 | ** | 3.1 ± 0.7 | | 2.7 ± 1.1 | | 2.7 ± 0.7 | ** | 3.0 ± 0.7 | * | 2.6 ± 0.7 | | 2.7 ± 0.6 | * | 2.8 ± 0.6 | ** | 2.9 ± 0.9 | |
| (mmol·L-1) | CID2 | 2.4 ± 0.6 | | 2.5 ± 0.7 | | 2.6 ± 0.8 | | 2.4 ± 0.7 | | 2.3 ± 0.4 | | 2.7 ± 0.6 | | 2.4 ± 0.3 | | 2.3 ± 0.7 | | 2.4 ± 0.8 | | 2.6 ± 0.8 | |
| Glucose | CID1 | 4.9 ± 0.8 | | 5.0 ± 0.6 | ** | 5.2 ± 0.7 | | 5.4 ± 0.9 | * | 4.7 ± 0.4 | | 4.8 ± 0.4 | | 4.8 ± 0.5 | | 5.0 ± 0.5 | | 4.8 ± 1.2 | | 5.0 ± 0.6 | |
| (mmol·L-1) | CID2 | 4.7 ± 0.7 | | 4.8 ± 0.4 | | 4.9 ± 1.0 | | 4.9 ± 0.6 | | 4.5 ± 0.4 | | 4.7 ± 0.4 | | 4.6 ± 0.5 | | 4.7 ± 0.4 | | 4.8 ± 0.5 | | 4.7 ± 0.3 | |
| HOMA-IR | CID1 | 2.0 ± 1.3 | *** | 2.4 ± 1.8 | *** | 2.4 ± 1.7 | ** | 3.8 ± 3.0 | ** | 1.8 ± 0.7 | ** | 1.7 ± 0.8 | * | 1.6 ± 0.7 | | 1.9 ± 0.8 | ** | 2.3 ± 1.8 | | 2.1 ± 0.7 | * |
| | CID2 | 1.2 ± 0.9 | | 1.4 ± 0.8 | | 1.3 ± 0.9 | | 1.8 ± 1.2 | | 1.0 ± 0.6 | | 1.1 ± 0.4 | | 1.1 ± 0.6 | | 1.1 ± 0.5 | | 1.5 ± 1.3 | | 1.5 ± 0.5 | |

Values are mean ± standard deviation from the fasted state. Bold values are different between successful and unsuccessful subjects by t-test (P<0.05).

BMI and body weight shows values from 3 CIDs, the means of different CIDs within a group without a common letter are different (P<0.001) by GLM repeated measures and Sidak post-hoc test. Others only show CID1 and CID2 values. Test for changes within a group is by paired t-test. CID2 v.s.CID1 P-value *: <0.05, **:<0.01, ***:<0.001.

EP 2 420 843 A1

Example 2: Measurement of ACE levels in plasma

ACE levels in plasma were analyzed by Rules Based Medicine, Inc 3300 Duval Road, Austin, TX 78759.

We measured ACE values on two time points before the diet intervention/weight maintenance, namely CID1 and CID2 (Table 4). Because age showed a trend to be significant for the difference between successful and unsuccessful weight maintenance, we corrected for age in the logistic models. We did not adjust for BMI or weight, because two third of the factors showed significant correlation with adiposity indices as expected.

Table 4: ACE levels in plasma in ng/ml at CID1 and CID2 (mean $\pm$ SD)

| | | | CID1 | CID2 | CID3 | CID2/CID1 |
|---|---|---|---|---|---|---|
| pool | Successful | N=48 | 159 $\pm$ 47 | 134 $\pm$ 41 | 148 $\pm$ 47 | 0,86 $\pm$ 0,15 |
| | Unsuccessful | N=48 | 153 $\pm$ 50 | 142 $\pm$ 45 | 155 $\pm$ 55 | 0,93 $\pm$ 0,11 |
| Diet1 LP/LGI | Successful | N=12 | 173 $\pm$ 43 | 144 $\pm$ 34 | 156 $\pm$ 35 | 0,86 $\pm$ 0,28 |
| | Unsuccessful | N=12 | 173 $\pm$ 59 | 157 $\pm$ 58 | 168 $\pm$ 54 | 0,91 $\pm$ 0,15 |
| Diet2 LP/HGI | Successful | N=12 | 144 $\pm$ 48 | 127 $\pm$ 42 | 142 $\pm$ 52 | 0,88 $\pm$ 0,06 |
| | Unsuccessful | N=12 | 132 $\pm$ 30 | 123 $\pm$ 28 | 142 $\pm$ 50 | 0,94 $\pm$ 0,08 |
| Diet3 HP/LGI | Successful | N=12 | 153 $\pm$ 50 | 128 $\pm$ 44 | 142 $\pm$ 48 | 0,84 $\pm$ 0,11 |
| | Unsuccessful | N=12 | 164 $\pm$ 43 | 150 $\pm$ 29 | 166 $\pm$ 40 | 0,93 $\pm$ 0,10 |
| Diet4 HP/HGI | Successful | N=12 | 166 $\pm$ 49 | 139 $\pm$ 43 | 151 $\pm$ 55 | 0,84 $\pm$ 0,08 |
| | Unsuccessful | N=12 | 143 $\pm$ 58 | 136 $\pm$ 56 | 144 $\pm$ 72 | 0,95 $\pm$ 0,10 |

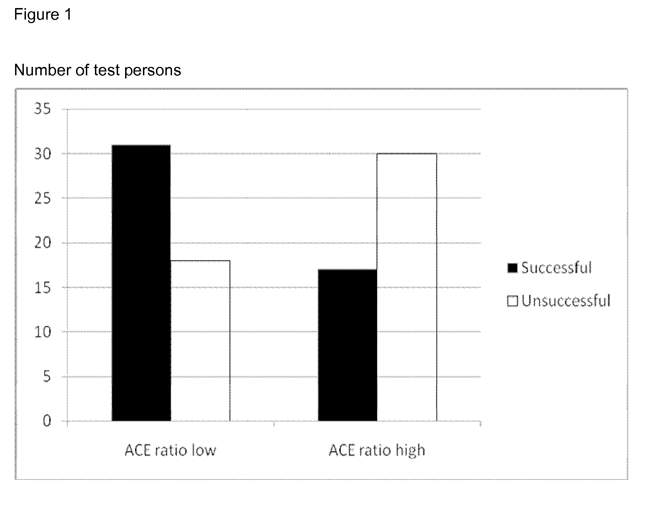

When we divided the groups of test persons into high and low ACE levels, we found that successful weight maintenance predominantly occurred in the group with the low ACE CID2/CID1 ratios. (Figure 1 and Table 5)

Table 5: Distribution of successful and unsuccessful outcome of weight maintenance in the group divided over high and low ACE CID2/CID1 ratios.

| | ACE ratio low | ACE ratio high |
|---|---|---|
| Successful | 31 | 17 |
| Unsuccessful | 18 | 30 |

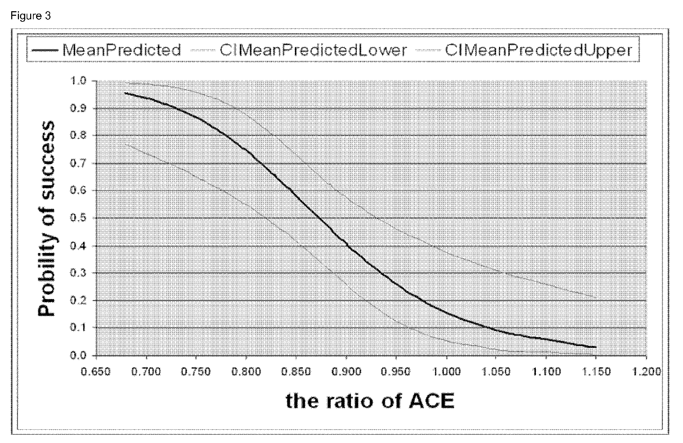

The probability of success of the method for predicting successful weight maintenance is plotted against the CID2/CID1 ratio of ACE in figure 3.

Example 3: Measurement of fibrinogen levels in plasma

Fibrinogen levels were determined at the onset of the study (CID1) by an in-house clotting assay of the diluted plasma in the presence of excess of thrombin using a STA® analyzer (Diagnostica Stago Inc, New Jersey, USA). The results are shown in table 2.

Example 4. Data analysis

Outliers defined as a data point disconnected from the others and out of the mean by $\pm$ 4 SD (p<0.0001) were removed. The anthropometric and physiological parameters were expressed as mean $\pm$ SD. Student t-test was applied to compare the existing difference of anthropometric and physiological parameters between successful and unsuccessful groups.

In the pooled subjects without taking the interaction between diet and blood proteins/steroids into account, the ratio of ACE (P=0.003), was significant to predict the outcome of weight maintenance.

Example 5: Fibrinogen levels in combination with ACE levels improve predictive value

Fibrinogen levels at the onset of the study were as such not predictive of successful or unsuccessful weight maintenance after weight loss (see Table 2). The test group of 96 individuals contained 51 individuals with a high fibrinogen level.

The term a "high fibrinogen level" means a fibrinogen level above a predetermined reference value. Preferably this reference value is obtained from a group of reference subjects comparable with respect to biometric measurements. Preferably, the reference value is the mean or median of the fibrinogen levels in these reference subjects. Preferably, the biometric measurements of the reference subjects include BMI or cholesterol levels.

Within this group of individuals with a high fibrinogen level, the CID2/CID1 ratio of ACE levels even more reliably predicted the outcome of successful weight maintenance. Hence, it appeared that ACE levels in combination with fibrinogen levels at the onset of the study reliably predicted whether weight loss was successful or not. This was even more reliable than the methods based on the ratio of ACE levels alone. The ACE ratios in the group of individuals with a high fibrinogen level are shown in table 1 and figure 2.

The positive predictive value of the method relying on ACE ratios only is 63%; 31 out of 49 individuals with a low ACE ratio were found to be successful in maintaining weight loss (Table 5). The positive predictive value of the method employing the levels of ACE as well as the levels of fibrinogen was 72%; 21 out of 29 individuals with high fibrinogen levels and low ACE levels were found to be successful in maintaining their weight loss.

References

1. Smyth S, Heron A 2006 Diabetes and obesity: the twin epidemics. Nat Med 12:75-80
2. Wing RR, Phelan S 2005 Long-term weight loss maintenance. American Journal of Clinical Nutrition 82:222S-225S
3. Moore CS, Lindroos AK, Kreutzer M, Larsen TM, Astrup A, van Baak MA, Handjieva-Darlenska T, Hlavaty P, Kafatos A, Kohl A, Martinez JA, Monsheimer S, Jebb SA 2010 Dietary strategy to manipulate ad libitum macronutrient intake, and glycaemic index, across eight European countries in the Diogenes Study. Obes Rev 11:67-75.
4. Larsen TM, Dalskov S, van Baak M, Jebb S, Kafatos A, Pfeiffer A, Martinez JA, Handjieva-Darlenska T, Kunesova M, Holst C, Saris WH, Astrup A 2010 The Diet, Obesity and Genes (Diogenes) Dietary Study in eight European countries - a comprehensive design for long-term intervention. Obes Rev 11:76-91.
5. Rankinen T, Zuberi A, Chagnon YC, Weisnagel SJ, Argyropoulos G, Walts B, Perusse L, Bouchard C 2006 The human obesity gene map: the 2005 update. Obesity (Silver Spring) 14:529-644
6. Qi L, Cho YA 2008 Gene-environment interaction and obesity. Nutrition Reviews 66:684-694
7. Vogels N, Mariman ECM, Bouwman FG, Kester ADM, Diepvens K, Westerterp-Plantenga MS 2005 Relation of weight maintenance and dietary restraint to peroxisome proliferator-activated receptor gamma 2, glucocorticoid receptor, and ciliary neurotrophic factor polymorphisms. American Journal of Clinical Nutrition 82:740-746
8. Phillips CM, Goumidi L, Bertrais S, Field MR, Peloso GM, Shen J, McManus R, Hercberg S, Lairon D, Planells R, Roche HM 2009 Dietary Saturated Fat Modulates the Association between STAT3 Polymorphisms and Abdominal Obesity in Adults. J Nutr 139:2011-2017
9. Goyenecha et al., 2006, British Journal of Nutrition 96, 965-972
10. Elfhag et al., 2005, Obesity reviews 6, 67-85.
11. Hill et al., 2009 Am. J. Clin. Nutr. 90; 276-281

**Claims**

1. Method for determining the probability that an individual will be successful in maintaining weight loss after an intentional weight loss by determining the level of angiotensin I converting enzyme (ACE) in a first sample obtained before the start of the weight loss and in a second sample obtained after the weight loss wherein the ratio of ACE levels in the second sample and the first sample is determined and compared to a predetermined first reference value and wherein it is concluded that the individual has a higher probability to be successful in maintaining weight loss if said ratio is below the predetermined reference value.

2. Method according to claim 1 wherein the first sample obtained from the individual has a fibrinogen level above a second predetermined reference value.

3. Method according to claims 1 or 2 wherein the sample is a blood, serum or plasma sample.

4. Method according to claims 1 - 3 wherein the intentional weight loss is due to a dietary period.

5. Method according to claims 1 - 4 wherein the first predetermined reference value is the mean ratio of CID2/CID1 of a population with comparable BMI and/or cholesterol levels.

6. Method according claims 1 - 5 wherein the second predetermined reference value is the mean or median of the fibrinogen levels in a population with comparable BMI and/or cholesterol levels.

Figure 1

Number of test persons

Figure 2

Number of test persons

Figure 3

| | Europäisches Patentamt |
|---|---|
| | European Patent Office |
| | Office européen des brevets |

**EUROPEAN SEARCH REPORT**

Application Number

EP 10 17 2846

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BUTKUS N E ET AL: "Angiotensin-converting enzyme activity decreases during fasting.", HORMONE AND METABOLIC RESEARCH = HORMON- UND STOFFWECHSELFORSCHUNG = HORMONES ET MÉTABOLISME FEB 1987 LNKD- PUBMED:3030914, vol. 19, no. 2, February 1987 (1987-02), pages 76-79, XP009143197, ISSN: 0018-5043 * Summary * | 1-6 | INV. G01N33/68 |
| A | KOSTIS JOHN B ET AL: "Association of angiotensin-converting enzyme DD genotype with blood pressure sensitivity to weight loss", AMERICAN HEART JOURNAL, vol. 144, no. 4, October 2002 (2002-10), pages 625-629, XP002616712, ISSN: 0002-8703 * Summary * | 1-6 | |
| A | HARP JOYCE B ET AL: "Dietary weight loss decreases serum angiotensin-converting enzyme activity in obese adults.", OBESITY RESEARCH OCT 2002 LNKD- PUBMED:12376578, vol. 10, no. 10, October 2002 (2002-10), pages 985-990, XP002616713, ISSN: 1071-7323 * abstract * | 1-6 | TECHNICAL FIELDS SEARCHED (IPC) G01N |

-/--

The present search report has been drawn up for all claims

| Place of search | Date of completion of the search | Examiner |
|---|---|---|
| Munich | 14 January 2011 | Mauhin, Viviane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 10 17 2846

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WEISINGER R S ET AL: "Angiotensin converting enzyme inhibition lowers body weight and improves glucose tolerance in C57BL/6J mice maintained on a high fat diet", PHYSIOLOGY AND BEHAVIOR, ELSEVIER SCIENCE LTD., OXFORD, GB, vol. 98, no. 1-2, 4 August 2009 (2009-08-04), pages 192-197, XP026266693, ISSN: 0031-9384, DOI: DOI:10.1016/J.PHYSBEH.2009.05.009 [retrieved on 2009-05-22] * abstract * | 1-6 | |
| A | MAVRI A ET AL: "Do baseline serum leptin levels predict weight regain after dieting in obese women?", DIABETES, OBESITY & METABOLISM AUG 2001 LNKD- PUBMED:11520310, vol. 3, no. 4, August 2001 (2001-08), pages 293-296, XP002616714, ISSN: 1462-8902 * Summary * | 1-6 | |

TECHNICAL FIELDS
SEARCHED      (IPC)

The present search report has been drawn up for all claims

| Place of search | Date of completion of the search | Examiner |
|---|---|---|
| Munich | 14 January 2011 | Mauhin, Viviane |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **SMYTH S ; HERON A.** Diabetes and obesity: the twin epidemics. *Nat Med,* 2006, vol. 12, 75-80
- **WING RR ; PHELAN S.** Long-term weight loss maintenance. *American Journal of Clinical Nutrition,* 2005, vol. 82, 222S-225S
- **MOORE CS ; LINDROOS AK ; KREUTZER M ; LARSEN TM ; ASTRUP A ; VAN BAAK MA ; HANDJIEVA-DARLENSKA T ; HLAVATY P ; KA-FATOS A ; KOHL A.** Dietary strategy to manipulate ad libitum macronutrient intake, and glycaemic index, across eight European countries in the Diogenes Study. *Obes Rev,* 2010, vol. 11, 67-75
- **LARSEN TM ; DALSKOV S ; VAN BAAK M ; JEBB S ; KAFATOS A ; PFEIFFER A ; MARTINEZ JA ; HANDJIEVA-DARLENSKA T ; KUNESOVA M ; HOLST C.** The Diet, Obesity and Genes (Diogenes) Dietary Study in eight European countries - a comprehensive design for long-term intervention. *Obes Rev,* 2010, vol. 11, 76-91
- **RANKINEN T ; ZUBERI A ; CHAGNON YC ; WEISNAGEL SJ ; ARGYROPOULOS G ; WALTS B ; PERUSSE L ; BOUCHARD C.** The human obesity gene map: the 2005 update. *Obesity (Silver Spring,* 2006, vol. 14, 529-644

- **QI L ; CHO YA.** Gene-environment interaction and obesity. *Nutrition Reviews,* 2008, vol. 66, 684-694
- **VOGELS N ; MARIMAN ECM ; BOUWMAN FG ; KESTER ADM ; DIEPVENS K ; WESTERTERP-PLANTENGA MS.** Relation of weight maintenance and dietary restraint to peroxisome proliferator-activated receptor gamma 2, glucocorticoid receptor, and ciliary neurotrophic factor polymorphisms. *American Journal of Clinical Nutrition,* 2005, vol. 82, 740-746
- **PHILLIPS CM ; GOUMIDI L ; BERTRAIS S ; FIELD MR ; PELOSO GM ; SHEN J ; MCMANUS R ; HERCBERG S ; LAIRON D ; PLANELLS R.** Dietary Saturated Fat Modulates the Association between STAT3 Polymorphisms and Abdominal Obesity in Adults. *J Nutr,* 2009, vol. 139, 2011-2017
- **GOYENECHA et al.** *British Journal of Nutrition,* 2006, vol. 96, 965-972
- **ELFHAG et al.** *Obesity reviews,* 2005, vol. 6, 67-85
- **HILL et al.** *Am. J. Clin. Nutr.,* 2009, vol. 90, 276-281